# EUROPEAN PATENT APPLICATION

(11) **EP 4 701 354 A1**
(43) Date of publication of application: **25.02.2026**
(21) Application number: 25196972.1
(22) Date of filing: 20.08.2025
(51) Int. Cl.: H05K 1/189, H05K 3/28, H05K 3/34, H05K 13/04, H05K 3/00, H05K 3/30, H05K 1/03, A61B 5/00, A61B 18/14, A61B 17/00, A61B 18/00, A61B 5/287

(54) **PLASTIC SLEEVE WITH EMBEDDED ELECTRODE AND FLEXIBLE PCB**

(30) Priority: 21.08.2024 US 202418810599
(71) Applicant: Biosense Webster (Israel) Ltd., 2066717 Yokneam (IL)
(72) Inventor: GOVARI, Assaf, 2066717 Yokneam (IL); ALTMANN, Andres Claudio, 2066717 Yokneam (IL); SITNITSKY, Ilya, 2066717 Yokneam (IL); DIUKMAN, Elad Avraham, 2066717 Yokneam (IL); SHECHTMAN, Alexander, 2066717 Yokneam (IL)
(74) Representative: Carpmaels & Ransford LLP

(57) **Abstract**

An apparatus for manufacturing catheter electrode-fPCB assemblies, the apparatus includes a tray, a heat source, and a sleeve molding station. The tray is configured to (i) receive an electrode in a designated first recess in a predefined layout of the recess, wherein the electrode is ring-shaped and (ii) receive a flexible printed circuit board (fPCB) strip in a second recess configured to enable to thread the fPCB strip via the electrode to a predefined position of the strip such that a pad patterned on the fPCB strip is aligned with the electrode. The heat source is configured to apply heat for soldering the pad to an inner surface of the electrode. The sleeve molding station is configured to mold an encapsulating sleeve over the electrode-fPCB assembly, while keeping a proximal end of the strip and at least a portion of the electrode exposed.

## Description

### FIELD OF THE DISCLOSURE

The present disclosure generally relates to the manufacturing of diagnostic and therapeutic catheters, and particularly to methods and apparatuses for establishing electrical connections for electrodes on these catheters.

### BACKGROUND OF THE DISCLOSURE

Certain catheters, such as those involved with cardiac mapping and ablating cardiac tissue, typically have multiple electrodes disposed over splines and electrically connected to a proximal end of the catheter. Multiple electrodes in a small space provide the catheter with precision and accuracy. Some catheters comprise ring-shaped electrodes, each manually soldered to a wire that may be subsequently connected to a multi-wire cable running along the shaft of the catheter to provide an electrical connection between each electrode and a connector at the proximal end of the catheter. The ring-shaped electrodes may be mounted on one or more splines, forming a distal end assembly of the catheter. The catheter is sized to fit through the vessels leading to the heart.

Currently, connecting such electrodes requires skilled personnel to perform tasks such as alignment and soldering. The small scale of the electrodes makes this process time-consuming, costly, and difficult to regulate for quality control.

The present disclosure will be more fully understood from the following detailed description of the examples thereof, taken together with the drawings, in which:

### BRIEF DESCRIPTION OF THE DRAWINGS

Fig. 1 is a schematic, pictorial illustration of a basket catheter assembly comprising ring electrodes mounted on splines, in accordance with an example of the present disclosure;
Fig. 2 is a schematic drawing of a tray comprised in an automated manufacturing apparatus (shown in Fig. 3) an electrode-fPCB assembly of a catheter, in accordance with an example of the present disclosure;
Figs. 3A-3C are schematic, pictorial illustrations of a first portion of the apparatus for automated manufacturing of an electrode-wire assembly of a catheter, and of an electrode-wire assembly in preparation, in accordance with an example of the present disclosure;
Figs. 4A and 4B are schematic, pictorial illustrations of a second portion of the apparatus for automated manufacturing of an electrode-wire assembly of a catheter, and a finished electrode-wire assemblies, in accordance with examples of the present disclosure; and
Fig. 5 is a flow chart that schematically illustrates a method for automatically manufacturing a catheter's electrode-fPCB assembly, in accordance with an example of the present disclosure.

### DETAILED DESCRIPTION OF EXAMPLES

### OVERVIEW

Catheters used in cardiac mapping and tissue ablation typically include multiple ring-shaped electrodes disposed over a distal end assembly. Some catheters include an expandable distal end assembly with multiple splines, e.g., a basket assembly. Each electrode on the distal end assembly needs to be connected electrically to a wire running in the catheter shaft. Conventionally, the connections are done manually in a labor-intensive process, as described above.

The present disclosure provides an automated (machine-based) manufacturing method and apparatus for electrically connecting the ring-shaped electrodes for each spline to a flexible printed circuit board (fPCB) and integrating the electrode-fPCB assembly on a sleeve. The sleeve may then be fitted onto a spline.

The disclosed automated manufacturing process connects all the electrodes to be mounted on a given spline to pads patterned on a distal portion of the fPCB strip. Each distal pad is electrically connected, independently, to a respective pad located at a proximal portion of the fPCB strip. In a subsequent automated process, the proximal pads of the fPCB are electrically connected to the wires running in the shaft.

The disclosed manufacturing technique includes forming a sleeve over the electrode-fPCB assembly using injection molding. This technique would be instead of mounting elements (e.g., fPCB strip and electrodes) on a prefabricated sleeve, e.g., off-the-shelf sleeve. In the later method, electrodes are typically connected to wires and mounted over the sleeve manually. In addition, the electrodes are required to be fixated onto the sleeve. The wall thickness of the sleeve is typically about 0.08-0.5 mm.

In one example, a manufacturing apparatus is provided that includes a tray, a roller, a mechanical z-stage, a heat source, and a sleeve molding device. The roller is configured to advance an fPCB strip onto the tray to thread the fPCB strip through each electrode to a predefined position of the strip such that pads patterned on the fPCB strip are aligned with the respective electrodes. The alignment can be achieved, for example, by having a predefined position of the strip dictated by the recess and advancing the strip to a predefined length. As another example, the alignment can be controlled by machine vision.

The z-stage lowers the electrodes, so contact is established between pads on the fPCB and an inner surface of the electrodes. The heat source is configured to apply heat to solder the pads to the electrodes. The sleeve molding device is configured to mold a sleeve over the electrode-fPCB assembly, keeping a proximal end of the strip and at least a portion of an outer surface (e.g., top outer facet) of the electrodes exposed. The remainder of the fPCB is configured to be embedded within the thickness of the sleeve wall.

In one example, the disclosed automated (machine-based) method for building electrode-fPCB assemblies includes the following steps:
1. Positioning ring-shaped electrodes in designated first recesses on a tray that holds the electrodes in a predefined layout (e.g., pitch).
2. Dispensing solder paste on the distal pads of the fPCB strip or using pre-tinned pads. Without loss of generality, the pads are assumed to be at the top side of the fPCB strip.
3. Sliding the fPCB strip through the hollow of the ring-shaped electrodes. The tray may have a second recess to direct the fPCB strip.
4. Pressing the PCB strip against an upper inner surface of the electrode by, for example, sliding a space holder (e.g., one formed with Teflon or Nitinol) under the fPCB strip, which presses it upward and against an inner facet of the electrode, thus creating contact between the electrode and the solder material.
5. Heating the electrodes, e.g., with a heating coil or blower, to solder the fPCB strip distal pads to the electrodes.
6. If step 4 uses another different pressing method than sliding a holder, then sliding now a space holder into the electrodes' hollow.
7. Placing the electrode-fPCB assembly in a molding device and over-molding injected plastic material (e.g., polyurethane or another flexible plastic) into a sleeve that embeds the electrode-fPCB assembly while maintaining the proximal end of the fPCB strip exposed and without fully covering the electrodes.
8. Pulling out the space holder leaving a hollow defined by the sleeve. The diameter of the hollow defined by the sleeve is within the same range of 0.08-0.5 mm.
9. Soldering wires to proximal pads of the fPCB strip that are exposed.

In subsequent manufacturing processes, the completed spline assemblies (made in steps 1-9) are assembled onto the catheter. This includes (i) sliding the sleeve of the prepared electrode-fPCB assembly over a spline of the catheter distal end and (ii) closing the catheter distal end into an expandable basket cage (e.g., by attaching the distal edges of the splines together to form a common catheter distal edge).

The wires soldered to the fPCB's proximal pads are then threaded (e.g., as a multi-wire cable) into the catheter's shaft.

The disclosed automated manufacturing process of steps 1-9 can be part of an automated manufacturing process for an entire multi-electrode catheter.

### CLINICAL SYSTEM DESCRIPTION

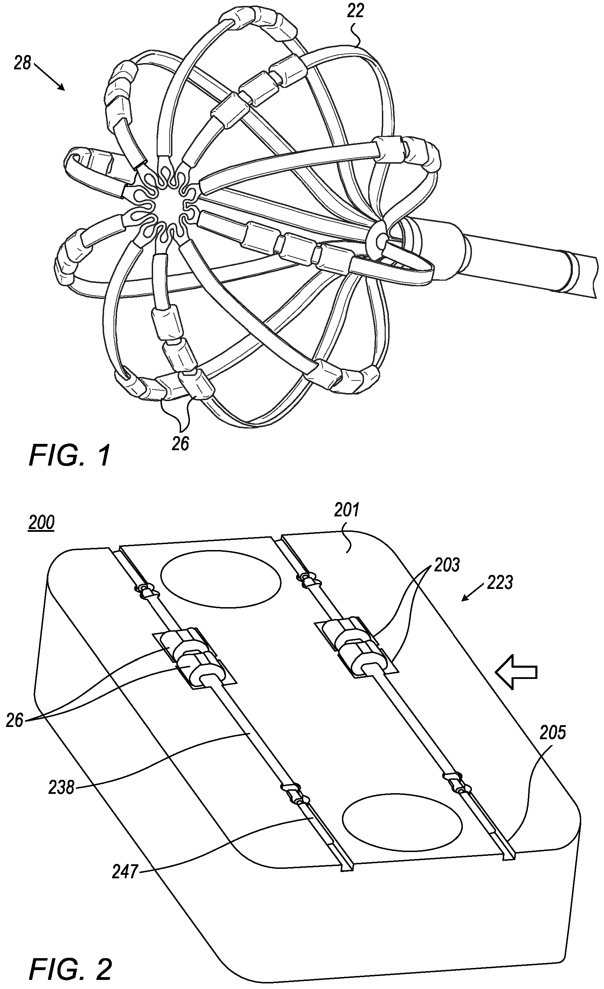

Fig. 1 is a schematic, pictorial illustration of a basket catheter assembly 28 comprising ring electrodes 26, in accordance with an example of the present disclosure. Basket catheter assembly 28 is formed by splines 22 mounted with electrodes 26. The present disclosure provides an automated (machine-based) method and apparatus for electrically connecting electrodes 26 for each of the splines 22 to an fPCB (shown in Fig. 2) and integrating the electrode-fPCB assembly on a sleeve. The sleeve may then be fitted onto a spline 22.

### POLYURETHANE SLEEVE WITH EMBEDDED ELECTRODE AND FPCB

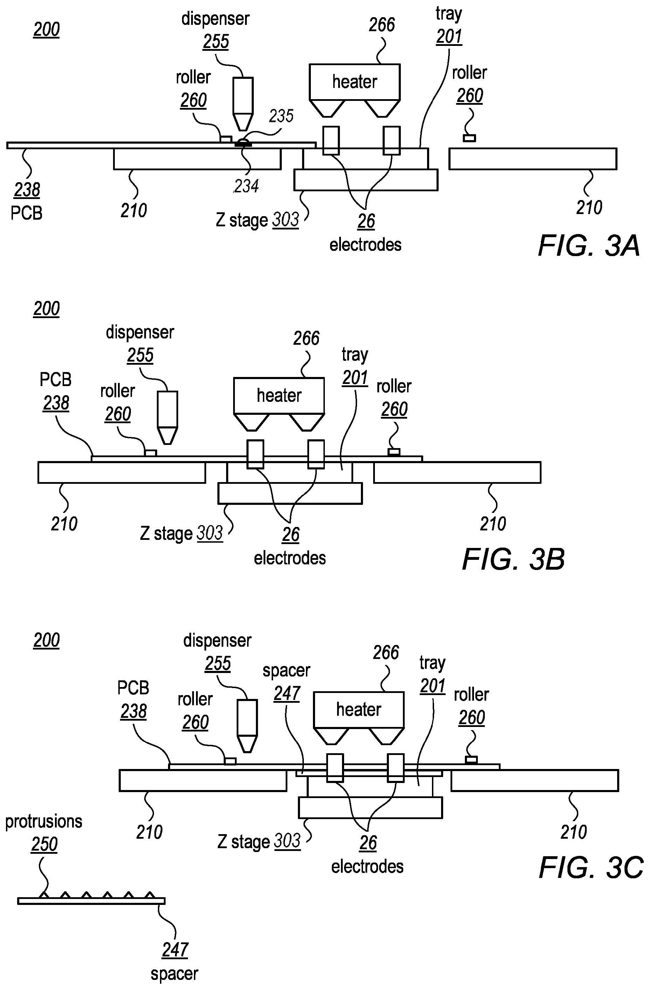

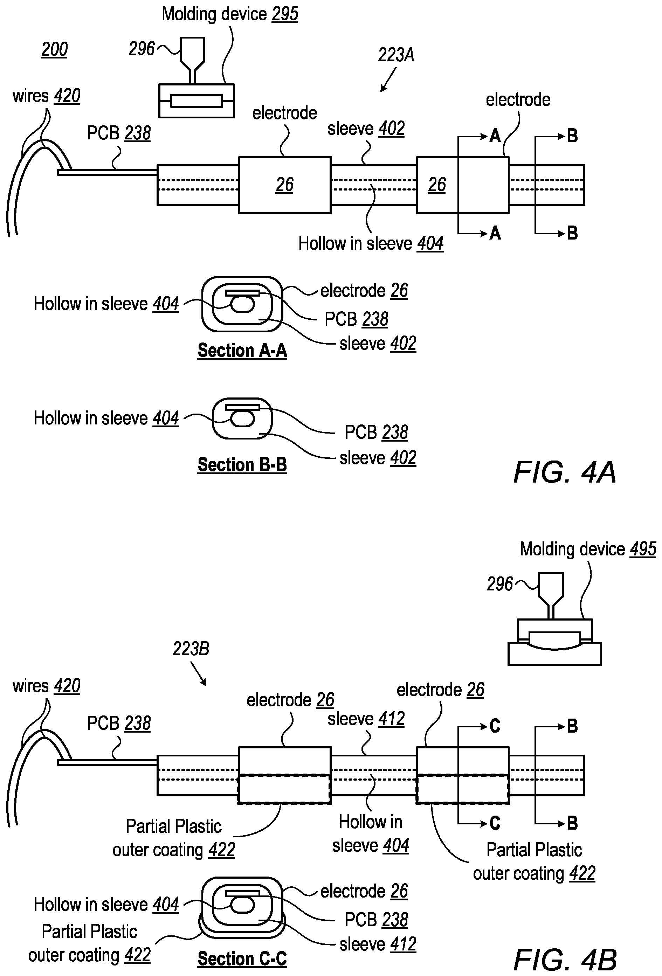

Fig. 2 is a schematic drawing of a tray 201 comprised in an automated manufacturing apparatus 200 (portions shown in Figs. 3 and 4) of an electrode-fPCB assembly 223 of a catheter, in accordance with an example of the present disclosure.

Fig. 2 shows parts of two electrode-fPCB assemblies 223 lying in tray 201 while manufacturing the electrode-fPCB assemblies. Electrodes 26 lay in designated first recesses 203 of tray 201 in a predefined layout. The tray includes a second recess 205 to assist in directing an fPCB strip 238 into a hollow of the electrodes, as described in Fig. 3. Optionally, the second recess 205 assists in directing a space holder 247, as also described below.

Figs. 3A-3C are schematic, pictorial illustrations of a first portion of apparatus 200 for automated manufacturing of an electrode-PCB assembly 223 of a catheter, and an electrode-PCB assembly 223 in preparation, in accordance with an example of the present disclosure.

Fig. 3A shows a solder paste dispensing station 255 dispenses solder paste 235 on a distal pad 234 of a fPCB strip 238 that lays on a left platform 210. Other distal pads (not shown) are dispensed with solder material after being advanced into a position under dispensing station 255 by a left roller 260.

As seen, two electrodes 26 lay in tray 201, which is supported by a z-stage 303.

In Fig, 3B, a roller 260 advanced PCB 238 through the hollow of electrodes 26 so that the pads 234 are aligned with the electrodes. The alignment can be achieved, for example, by having a predefined position of strip 238 dictated by the recess 205 and roller 260, advancing the strip to a predefined length. As another example, the alignment can be controlled by machine vision.

In Fig. 3C, z-stage 303 lowers tray 201, thereby bringing an inner surface of electrodes 26 into contact with the paste 235 on pads 234 of the fPCB.

Optionally, a spacer 247 having protrusions 250 is inserted under the fPCB to press fPCB 238 upward, against an inner facet of each electrode 26.

A heat source 266 (e.g., a heating coil or a blower) applies heat to solder pads 234 to electrodes 26.

Finally, a right roller 260 pulls the soldered electrode-PCB assembly to a right platform 210.

### ELECTRODE-FPCB ASSEMBLY ENCAPSULATION SCHEMES BY MOLDING

### A SLEEVE

Figs. 4A and 4B are schematic, pictorial illustrations of a second portion of apparatus 200 for automated manufacturing of an electrode-wire assembly 223 of a catheter, and finished electrode-wire assemblies 223, in accordance with examples of the present disclosure.

Figs. 4A and 4B show, respectively, versions 223A and 223B of electrode-wire assembly 223. The only difference between versions 223A and 223B is the sleeve configuration at electrodes 26, as described below.

In Fig. 4A, a molding device 295 over-molds an injected sleeve material 296 into a sleeve 402 over and around electrode-fPCB strip assembly 223A, keeping the proximal end of fPCB strip 238 exposed so it can be wired (420).

Section A-A and section B-B show both a hollow 404 in sleeve 402 (to let a subsequent threading of the electrode-fPCB assembly 223 over spline 22).

As seen, fPCB strip 238 is embedded in the wall of the molded sleeve 402, with the isolated backside of strip 238 exposed by hollow 404. In other options, such as with a space 247 with protrusions 250 or by lifting strip 238 at its edges, fPCB strip 238 may be fully embedded in the sleeve's wall.

As section A-A further shows, the over-molding of sleeve 402 leaves all external facets of electrode 26 exposed. In other examples, the over-molding of the sleeve leaves at least the top outer surface of the electrode exposed.

Fig. 4B shows the over-molding of sleeve 412 using another cast (495) of the molding device. With cast 495, as section C-C shows, a sleeve 412 portion 422 covers at least the back surface of electrode 26, i.e., the surface that faces the basket catheter assembly 28 inner volume. This way an electric field generated between adjacent electrodes mostly extends outside basket 28. The covering of the backside of the electrodes can reduce, for example, ablation current via the blood pool of a cardiac chamber.

Figs. 3 and 4 are presented as an example only to demonstrate the principles of the disclosed technique without burdening the description by showing many manufacturing devices (such as mechanized arms) used in an automated process.

### METHOD OF MANUFACTURING A PLASTIC SLEEVE WITH EMBEDDED ELECTRODE AND FPCB

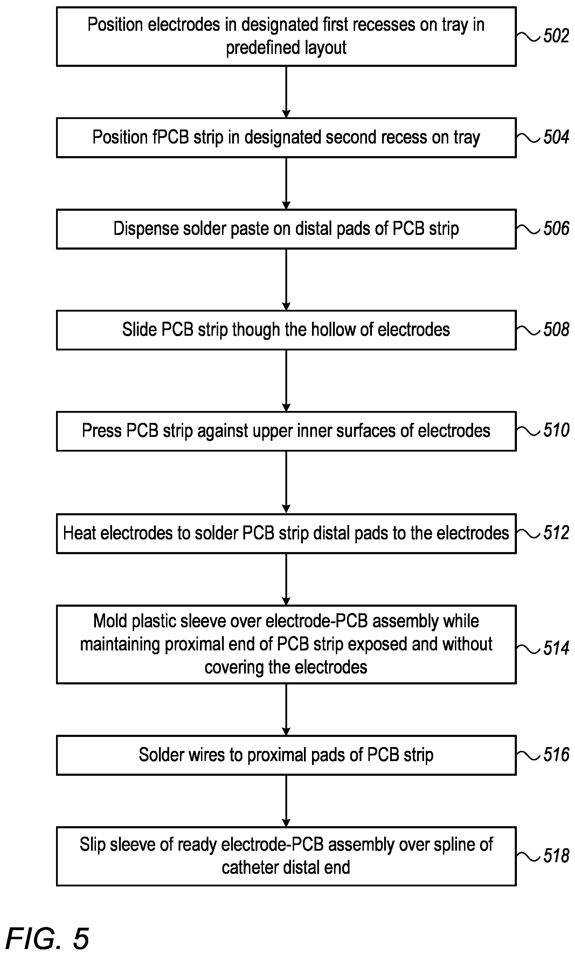

Fig. 5 is a flow chart that schematically illustrates a method for manufacturing a catheter's electrode-fPCB assembly 223 in an automated process, in accordance with an example of the present disclosure.

The method, according to the presented example, carries out a process that begins at electrodes positioning step 502, during which, for example, a user manually places electrodes 26 in designated first recesses 203 on a tray 201. The tray holds the electrodes at the recesses' predefined layout (e.g., pitch). Optionally, an automated arm may position the electrodes.

At fPCB strip positioning step 504, fPCB strip 238 is placed on left platform 210 in a designated second recess 205.

At solder material dispensing step 506, solder paste dispenser 255 dispenses solder paste 235 on distal pads 234 of fPCB strip 238 (or, alternatively, pre-tinned pads are used). Without loss of generality, pads 234 are assumed to be located on the top side of the fPCB strip.

Roller 260 may be used to advance fPCB strip 238 in second recess 205 and through the hollow of the electrodes, at fPCB strip sliding step 508.

At fPCB pressing step 510, fPCB strip 238 is pressed against the upper inner surfaces of the electrodes. One way of doing this is to slide space holder 247 (e.g., formed with heat-resilient Teflon or Nitinol) under the fPCB strip. This presses the fPCB upward against an inner facet of the electrode to create contact between the electrodes and the solder material.

At a joining step 512, heat source 266 applies heat to join pad on fPCB to electrode with solder material.

At molding step 514, an injection molding device 295 molds a sleeve 250 over the electrode-fPCB assembly 223 while maintaining the proximal end of the fPCB strip exposed and without covering electrodes 26.

If a space holder 247 was used, then a user pulls it out (e.g., retracts). Alternatively a mechanized arm may be used for pulling space holder 247.

The electrode-fPCB assembly 223 is completed by soldering wires to the proximal pads of fPCB strip 238, at a second soldering step 516.

Finally, the sleeve of the prepared electrode-fPCB assembly 223 may be slipped over a spline 22 of the catheter distal end 28 and fixed to the spline, at spline assembly step 518.

The flowchart in Fig. 3 is used as an example. Alternative steps, such as manually performing step 518, may apply. Different manufacturing tooling, such as grippers and a welder for wiring the proximal pads in step 512, may also be used.

### EXAMPLES

### Example 1

An apparatus (200) for manufacturing catheter electrode-fPCB assemblies (223), the apparatus includes a tray (201), a heat source (266), and a sleeve molding station (295). The tray (201) is configured to (i) receive an electrode (26) in a designated first recess (203) in a predefined layout of the recess, wherein the electrode (26) is ring-shaped and (ii) receive a flexible printed circuit board (fPCB) strip (238) in a second recess (205) configured to enable to thread the fPCB strip via the electrode to a predefined position of the strip such that a pad (234) patterned on the fPCB strip (238) is aligned with the electrode (26). The heat source (266) is configured to apply heat for soldering the pad (234) to an inner surface of the electrode (26). The sleeve molding station (295) is configured to mold an encapsulating sleeve (402, 412) over the electrode-fPCB assembly, while keeping a proximal end of the strip (238) and at least a portion of the electrode (26) exposed.

### Example 2

The apparatus (200) according to example 1, wherein the sleeve molding station (295) is configured to leave at least a top outer facet of the electrode (26) exposed.

### Example 3

The apparatus (200) according to example 1, wherein the sleeve molding station (295) is configured to cover at least a back surface of the electrode (26).

### Example 4

The apparatus (200) according to any of examples 1 through 3, further comprising a roller (260) configured to advance the fPCB strip (238) in the second recess (205) in the tray (201) to thread the fPCB strip through the electrode (26) to the predefined position.

### Example 5

The apparatus (200) according to any of examples 1 through 4, further comprising a mechanical z-stage (303) configured to lower the electrode (26) so contact is established between the pad (234) on the fPCB strip (238) and an inner surface of the electrode.

### Example 6

The apparatus (200) according to any of examples 1 through 5, wherein the heat source (266) is configured to solder the pad (234) by heating a solder paste (235) that was dispensed beforehand on the pad using a paste dispenser (255).

### Example 7

The apparatus (200) according to any of examples 1 through 6, wherein the pad (234) is pre-tinned, and wherein the heat source (266) is configured to solder the pad by heating the pre-tinned pad.

### Example 8

The apparatus (200) according to any of examples 1 through 7, wherein the heat source (266) comprises a hot air blower.

### Example 9

The apparatus (200) according to any of examples 1 through 8, wherein the station (295) comprises a mold positioned over the assembly (223) for performing injection over molding.

### Example 10

The apparatus (200) according to any of examples 1 through 9, further comprising machine vision to align the fPCB pad with electrode.

### Example 11

A method for manufacturing catheter electrode-fPCB assemblies (223), the method comprising receiving an electrode (26) in a tray (201) in a designated first recess (203) in a predefined layout of the recess, wherein the electrode (26) is ring-shaped. A flexible printed circuit board (fPCB) strip (238) is received in the tray (201) in a second recess (205). The fPCB strip (238) is threaded via the electrode along the second recess (205) to a predefined position of the strip such that a pad (234) patterned on the fPCB strip (238) is aligned with the electrode (26). Using a heat source (266), heat is applied for soldering the pad (234) to an inner surface of the electrode (26). Using a sleeve molding station (295), an encapsulating sleeve (402, 412) is molded over the electrode-fPCB assembly, while keeping a proximal end of the strip (238) and at least a portion of the electrode (26) exposed.

Although the examples described herein mainly address cardiac diagnostic applications, the methods and apparatuses described herein can also be used in other medical applications.

It will be appreciated that the examples described above are cited by way of example, and that the present disclosure is not limited to what has been particularly shown and described hereinabove. Rather, the scope of the present disclosure includes both combinations and subcombinations of the various features described hereinabove, as well as variations and modifications thereof which would occur to persons skilled in the art upon reading the foregoing description and which are not disclosed in the prior art.

## Claims

1. An apparatus (200) for manufacturing catheter electrode-fPCB assemblies (223), the apparatus comprising:
a tray (201) configured to (i) receive an electrode (26) in a designated first recess (203) in a predefined layout of the recess, wherein the electrode (26) is ring-shaped and (ii) receive a flexible printed circuit board (fPCB) strip (238) in a second recess (205) configured to enable to thread the fPCB strip (238) via the electrode to a predefined position of the strip such that a pad (234) patterned on the fPCB strip is aligned with the electrode (26);
a heat source (266) configured to apply heat for soldering the pad (234) to an inner surface of the electrode (26); and
a sleeve molding station (295) configured to mold an encapsulating sleeve (402, 412) over the electrode-fPCB assembly, while keeping a proximal end of the strip and at least a portion of the electrode (26) exposed.

2. The apparatus (200) according to claim 1, wherein the sleeve molding station (295) is configured to leave at least a top outer facet of the electrode (26) exposed.

3. The apparatus (200) according to claim 1, wherein the sleeve molding station (295) is configured to cover at least a back surface of the electrode (26).

4. The apparatus (200) according to any of claims 1 to 3, further comprising a roller (260) configured to advance the fPCB strip (238) in the second recess (205) in the tray (201) to thread the fPCB strip through the electrode (26) to the predefined position.

5. The apparatus (200) according to any preceding claim, further comprising a mechanical z-stage (303) configured to lower the electrode (26) so contact is established between the pad (234) on the fPCB strip (238) and an inner surface of the electrode.

6. The apparatus (200) according to any preceding claim, wherein the heat source (266) is configured to solder the pad (234) by heating a solder paste (235) that was dispensed beforehand on the pad using a paste dispenser (255).

7. The apparatus (200) according to any preceding claim, wherein the pad (234) is pre-tinned, and wherein the heat source (266) is configured to solder the pad by heating the pre-tinned pad.

8. A method for manufacturing catheter electrode-fPCB assemblies (223), the method comprising:
receiving an electrode (26) in a tray (201) in a designated first recess (203) in a predefined layout of the recess, wherein the electrode (26) is ring-shaped;
receiving in the tray in a second recess a flexible printed circuit board (fPCB) strip (238);
threading the fPCB strip (238) via the electrode along the second recess (205) to a predefined position of the strip such that a pad (234) patterned on the fPCB strip (238) is aligned with the electrode (26);
using a heat source (266), applying heat for soldering the pad (234) to an inner surface of the electrode (26); and
using a sleeve molding station (295), molding an encapsulating sleeve (402, 412) over the electrode-fPCB assembly, while keeping a proximal end of the strip (238) and at least a portion of the electrode (26) exposed.

9. The method according to claim 8, wherein molding the sleeve comprises leaving at least a top outer surface of the electrode (26) exposed.

10. The method according to claim 8, wherein molding the sleeve comprises covering at least a back surface of the electrode (26).

11. The method according to any of claims 8 to 10, further comprising, using a roller (260), advancing the fPCB strip (238) in the second recess (205) in the tray (201) to thread the fPCB strip through the electrode (26) to the predefined position.

12. The method according to any of claims 8 to 11, further comprising, using a mechanical z-stage (303), lowering the electrode (26) so contact is established between the pad (234) on the fPCB strip (238) and an inner surface of the electrode.

13. The method according to any of claims 8 to 12, wherein heating to solder the pad (234) comprises heating a solder paste (235) that was dispensed beforehand on the pad using a paste dispenser (255).

14. The method according to any of claims 8 to 13, wherein the pad (234) is pre-tinned, and wherein soldering the pad comprises heating the pre-tinned pad.

15. The apparatus (200) according to any of claims 1 to 7, or the method according to any of claims 8 to 14, wherein the heat source (266) comprises a hot air blower.

16. The apparatus (200) according to any of claims 1 to 7 or 15, or the method according to any of claims 8 to 15, wherein the station (295) comprises a mold positioned over the assembly (223) for performing injection over molding.

17. The apparatus (200) according to any of claims 1 to 7, 15 or 16, further comprising machine vision to align the fPCB pad with electrode (26).

18. The method according to any of claims 8 to 16, wherein aligning the fPCB pad with the electrode (26) comprises using machine vision.
